# EUROPEAN PATENT APPLICATION

(11) **EP 1 466 663 A1**
(43) Date of publication of application: **13.10.2004**
(21) Application number: 04006597.1
(22) Date of filing: 18.03.2004
(51) Int. Cl.: B01J 19/00, B01J 19/08, B01J 19/10, B01J 19/12, C12Q 1/68, C07H 19/16, C07H 19/06, C07H 19/10, C07H 19/20, C07H 21/00

(54) **Quality control method for manufacturing nucleic acid arrays**

(30) Priority: 19.03.2003 EP 03006098
(71) Applicant: F. HOFFMANN-LA ROCHE AG, 4070 Basel (CH); Roche Diagnostics GmbH, 68305 Mannheim (DE)
(72) Inventor: Mauritz, Ralf, Dr., 82377 Penzberg (DE); Heindl, Dieter, Dr., 82396 Pähl (DE)
(74) Representative: Weiss, Wolfgang, Dipl.-Chem. Dr.

(57) **Abstract**

The invention relates to a quality control method for manufacturing biopolymer arrays comprising the use of detectable protecting groups.

## Description

The invention relates to a quality control method for manufacturing biopolymer arrays comprising the use of detectable protecting groups.

The synthesis of nucleic acids and peptides on a solid phase has been an established process for the last 20 years. The most prevalent method of nucleic acid synthesis is the phosphoramidite method of Beaucage and Caruthers, Tetrahedron Lett. 22 (1981), 1859-1862, where the oligonucleotide chain is built up by the repetitive condensation of individual nucleotide building blocks in the 3' or 5' direction. A variety of orthogonal protecting groups are used to protect three reactive nucleotide groups: the ribose sugar 5' hydroxyl group, the amino protecting group of the nucleobases adenine, guanine and cytosine (thymine does not need a protecting group), as well as the phosphate protecting group of the nucleotide 3' phosphate residue. These protecting groups are then cleaved off under varying conditions, either during or after the synthesis. The 4,4'-dimethoxytriphenylmethyl (DMT) group has become the standard for 5' hydroxyl, the 2-cyanoethyl group the standard for phosphate residue and various acyl groups the standard for the amino functions of the nucleobases according to Büchi and Khorana (J. Mol. Biol. 72 (1972), 251-258) and Souveaux (in: "Methods in Molecular Biology" Vol. 26, Chap. 1 Protocol for Oligonucleoside Conjugates, Ed. S. Agrawal, Humana Press Inc., Totowa, N.J. 1994). The DMT group is cleaved off during synthesis in order to generate an hydroxyl group to which the next phosphoramidite can bind. The other named protecting groups remain until the end of the synthesis in order to prevent any side-reactions or by-products. At the end of the synthesis the complete oligonucleotide is completely deprotected largely by means of a base, whereby 2-cyanoethyl and acyl protecting groups are cleaved off.

There are essentially two means of producing biochips: off-chip and on-chip synthesis of oligonucleotide probes. In off-chip synthesis, the oligonucleotide is produced on a commercially available synthesizer using the above-mentioned standard reagents and then immobilized on the chip. In on-chip synthesis, the oligonucleotide is produced directly on the chip using the above-mentioned standard reagents. In the former case, the quality of the oligonucleotide can be analysed by means of analytical processes such as HPLC or mass spectrometry and, where necessary, improved via purification. The later case of on-chip synthesis allows for only limited quality control and purification is not possible. Quality control is normally only possible by means of the covalent binding of a (mainly fluorescent) colouring material at the terminus of the oligonucleotide, which can then be detected and quantified.

There is scarcely any mention in the literature of processes for determining the deprotection degree of oligonucletides. A first method comprises the detection of nucleobases and 5'-hydroxyl protecting groups on oligonucleotides with monoclonal antibodies, Fu et al., Analytical Biochemistry (2002), 306( 1 ), 135-143). Other references describe the use of cleavable, fluorescent protecting groups. These refer, however, to protecting groups for the 5'-hydroxyl group or the phosphate residue, but not for the nucleobase amino groups. In this context, it should be noted that US Patent 6,238,862 B1 describes a fluorescent, photo labile protecting group for the 5'-hydroxyl group used for determining the synthesis efficiency of the DNA synthesis array. Wagner and Pfleiderer (Helv. Chim. Act. 80 (1990), 200-212) describe a nucleobase protecting group which basically has fluorescent properties. However, this group has been developed for other purposes, especially for improved deprotection properties by using a β-elimination reaction process.

As a rule, exact quality controls cannot be carried out with on-chip synthesis. Presently, only the binding of dyes to the terminus of the oligonucleotide gives an indirect indication of the quantity of solid phase oligonucleotide and of the quality of the synthesis. This does not, however, provide any indication of the rate of the deprotection at the end of the synthesis, i.e. whether all 2-cyanoethyl and acyl protecting groups were cleaved off. It is known that, under standard conditions, the nucleobase acyl protecting groups cannot always be quantitatively cleaved off and part of the amino groups therefore remain protected. Complete deprotection of the amino groups is however essential for the optimal application of the chip. Should not all of the amino function protecting groups be cleaved off, a subsequent hybridisation can be decisively impaired, since the formation of Watson-Crick or Hoogsteen base pairs will be inhibited.

Thus, the invention relates to a quality control method for manufacturing biopolymer arrays comprising:
(a) synthesizing a plurality of different biopolymer species on an array from monomeric or oligomeric building blocks comprising detectable protecting groups;
(b) optionally carrying out a determination of the detectable protecting groups on the array after synthesis;
(c) cleaving off the detectable protecting groups, and
(d) carrying out a determination of the detectable protecting groups on the array after cleavage; in order to determine the efficacy of deprotection.

These simple steps would enable the quality control of the synthesised biopolymers. The main focus is quality control of the completeness of the biopolymer deprotection. The key advantage is that the methodology is non-destructive and requires no further steps once the final deprotection or detection has been carried out. The outcome is an evaluation of the rate of deprotection as well as of the quality of freely accessible biopolymers for later use.

Generally, the invention relates to the use of detectable protecting groups in the manufacture of biopolymer arrays. The term "biopolymer" as used in the present application particularly relates to nucleic acids such as DNA or RNA or nucleic acid analogues such as peptide nucleic acids (PNA) or locked nucleic acids (LNA) or combinations thereof. The term, however, also relates to peptides and peptide analogues as well as to other biopolymers such as carbohydrates or any combinations thereof. Preferably the biopolymer species are selected from nucleic acids and nucleic acid analogues wherein the detectable protecting groups are coupled to nucleobases, particularly to amino groups of nucleobases.

The term "array" as used in the present application relates to a solid support e.g. a planar or non-planar solid support. The solid support may have a surface selected from materials such as silicon, metal, metal oxides such as silica, glass or plastic or any combination thereof. Preferably the array is a chip.

The method of the present invention comprises synthesizing a plurality of different biopolymer species on the array from monomeric or oligomeric building blocks. For example, nucleic acids may be synthesized from phosphoamidite or phosphonate building blocks as known in the art. The synthesis is a spatially directed synthesis, e.g. a synthesis of different biopolymer species on different locations on the carrier. Methods for spatially directed biopolymer synthesis include, without limitation, light-directed synthesis, microlithography, application by inkjet, pin printing, microchannel deposition to specific locations and sequestration with physical barriers. In general, these methods involve generating active sites on the carrier, usually by removing protecting groups and coupling to the active site a monomeric or oligomeric building block which, itself, optionally has a protected active site if further coupling of building blocks is desired.

In a preferred embodiment, the synthesis of the biopolymer species is carried out in a manner that the detectable protecting groups remain on the biopolymer species until the synthesis has been terminated. It should be noted, that the synthesis can be carried out using standard protocols.

After synthesis has been terminated a first determination of the detectable protecting groups on the array may be carried out. By this means, qualitative and/or quantitative determination of the biopolymer species on the array is possible. The first determination step is preferably a spatially resolved determination step wherein a qualitative and/or quantitative determination of detectable protecting groups is carried out separately on different locations of the array. Techniques for spatially directed detection procedures may comprise the use of spatially resolved detectors e.g. microscopes or detector matrices such as CCD imaging systems allowing a parallel determination on a plurality of locations on the array.

After the optional first determination the detectable protecting groups are cleaved off. The cleavage may be carried out by known protocols according to the nature of the respective detection groups e.g. by photochemical methods such as irradiation, or by chemical methods such as acid or base treatment. In this context it should be noted that the detectable protecting groups are preferably selected such that they are not cleaved off during the biopolymer synthesis procedure.

After the cleavage of the detectable protecting groups a determination of the detectable protecting groups is carried out on the array in order to determine the efficacy of the protection. This determination may be qualitative and/or quantitative. If a first determination is carried out, the difference between the amount of protecting groups before and after cleavage is an indication of the amount of deprotected biopolymer and thus an indication of the deprotection rate. When no more protecting groups are detected after deprotection the deprotection has been quantitative, otherwise a repetition of deprotection may be necessary.

In a first embodiment of the invention the detectable protecting groups are selected from fluorescent groups, e.g. groups comprising pyrene, dansyl, stilbene, rhodamine and/or coumarine moieties. An important feature of the fluorescent groups is that they are stable during oligonucleotide synthesis. For example, the fluorescent moiety may be combined with an acyl, e.g. a tert-butylphenoxyacetyl protecting group or another amino protecting group.

In a further embodiment the detectable protecting groups may be selected from radioactively detectable protecting groups. For example, radioactively detectable groups are selected from groups comprising ¹⁴C, ³²P and ³H doped moieties which may be combined with a suitable protecting group, e.g. an amino protecting group.

In a still further embodiment the detectable protecting groups may be selected from electrochemically detectable protecting groups, e.g. groups comprising ferrocene and/or phenothiazine moieties. These moieties may be combined with suitable protecting groups, e.g. amino protecting groups. The determination of such protecting groups may be carried out by electrochemical methods, e.g. voltammetry.

In a still further embodiment of the present invention the detectable protecting groups may be selected from UV or IR detectable protecting groups, e.g. from groups comprising aromatic nitro moieties, hydroxyl moieties, thiole, thioether or thiophenole moieties, nitrile moieties, isocyanate moieties and/or halo moieties. UV and IR detectable protecting groups are preferably detectable independently from the biopolymer species which has been synthesised on the array.

In a still further embodiment the detectable protecting groups are selected from bioaffine detectable protecting groups. Bioaffine detectable protecting groups comprise moieties selected from partners of a bioaffine binding pair which can be detected by their specific interaction with the respective other partner of the binding pair. Specific examples of bioaffine detectable protecting groups are protecting groups comprising biotin, digoxin or digoxigenin moieties. Biotin may be detected by its bioaffine interaction with streptavidin or avidin or with anti-biotin-antibodies. Digoxin or digoxigenin may be detected by their specific interaction with respective antibodies.

In an especially preferred embodiment of the present invention the building blocks for the biopolymer synthesis are nucleotide building blocks having the general structural formulae I or II: 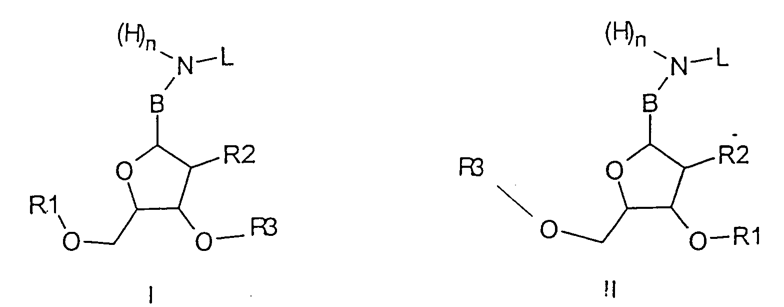 wherein R¹ is an hydroxy protecting group,
R² is -H, -(C₁-C₁₀)-alkoxy, -(C₂-C₁₀)-alkenyloxy, -(C₂-C₁₀)-alkynyloxy, -halogen, -azido, -NHR7, -SR7 or -OR7, wherein R7 is a protecting group or a reporter group,
R³ is a phosphate, an H-phosphonate or other phosphate analogue group which may contain a protecting group,
B is a nucleobase or a nucleobase analogue,
n is 0 or 1, and
L is a detectable protecting group, e.g. of the structure -C(O)-R, when n = 1, or = CH-NR⁸R, when n = 0, wherein R is the residue of the reporter group and R⁸ is selected from the group consisting of -(C₁-C₃)-alkyl.

In the compounds (I) or (II) the 5' or 3' hydroxy protecting group R¹ is preferably selected from optionally substituted triphenylmethyl groups, e.g. 4,4'-dimethoxy triphenylmethyl or 4-monomethoxy triphenylmethyl, pixyl groups, photocleavable groups, e.g. p-nitrophenylpropoxy carbonyl (NPPOC) or (α-methyl)-6-nitropiperonyloxy carbonyl (MeNPOC), and substituted silyl protecting groups, e.g. tert-butyldimethyl silyl (TBDMS) or tert-butyldiphenyl silyl (TBDPS).

The group R³ is a phosphate or phosphate analogue group which may contain a protecting group, preferably a phosphitamide group, more preferably a group -P(R⁶)NR4R5, wherein R⁴ and R⁵ are independently selected from the group consisting of -H, -(C₁-C₁₀)-alkyl, -(C₂-C₁₀)-alkenyl, -(C₆-C₂₂)-aryl, or wherein NR4R5 can form together with N a 5-6-membered heterocyclic ring,
R⁶ is selected from the group consisting of -(C₂-C₆)-alkenyloxy, -(C₂-C₆) alkenyl, -(C₁-C₆)-alkyl, or -(C₁-C₆)-alkoxy,
wherein each group may contain one or several substituents selected from -halo, p-nitroaryloxy and -cyano or wherein R⁶ is -H.
R⁶ is most preferably a 2-cyanoethyloxy group.

B is a nucleobase or nucleobase analogue having at least one amino group, e.g. adenine, guanine, cytosine, or a corresponding nucleobase analogue. The nucleobase or nucleobase analogue is preferably capable of hydrogen bridge formation with a complementary nucleobase after incorporation into a nucleic acid molecule. Suitable nucleobase analogues are mono- and bicyclic heterocycles comprising at least one amino group wherein the heterocycle is different from natural nucleobases as described by Simons, (Advanced 2001 Chemistry Texts, Nucleoside Mimetics, Gordon and Breach Science Publishers, Amsterdam 2001, Chapter 4), for example aza analogues of naturally occurring nucleobases, wherein a CH-moiety of a purine or a pyrimidine ring is replaced by nitrogen (such as 8-aza-adenosine) or deaza analogues of naturally occurring nucleobases wherein an N atom in the ring is substituted by a CH group (such as 7-deaza-guanosine) or combinations of aza and deaza substitutions (such as 8-aza-7-deaza-guanosine). Further preferred nucleobase analogues are C-nucleosides, e.g. deaza nucleobases wherein the N9 atom of a purine base or the N1 atom of a pyrimidine base respectively is substituted by a carbon atom (in the sp² configuration) such as formycine and pseudoisocytidine. Further, suitable nucleobase analogues may carry additional amino groups, e.g. 2-aminoadenosine. Further, the nucleobase or nucleobase analogue may be substituted, wherein the substitutent, e.g. a C₁-C₃ alkyl or alkoxy, a C₂-C₃ alkenyl or alkenyloxy, a C₂-C₃ alkynyl or alkynyloxy and/or a halo substituent, is preferably compatible with the formation of hydrogen bridges to a complementary base. Preferred positions for substituents are C5 in pyrimidine bases, C8 in purine bases and C7 in deaza purine bases.

Furthermore, the present invention relates to a nucleic acid synthesis building block having the general structural formulae I or II as described above.

The building blocks are suitable for the production of nucleic acid arrays.

Furthermore, the invention relates to a reagent kit for the synthesis of nucleic acid arrays comprising at least one nucleic acid synthesis building block as described above. For example, the reagent kit may comprise at least two different nucleic acid synthesis building blocks each carrying different detectable protecting groups. The different detectable protecting groups are preferably detectable independent from each other. For example, the reagent kit may comprise at least two different independently detectable fluorescent protecting groups, or combinations of different embodiments of detectable protecting groups, e.g. fluorescent and bioaffine detectable protecting groups or other combinations of different protecting groups.

Further, the invention shall be explained in more detail by the following Figures and Example.

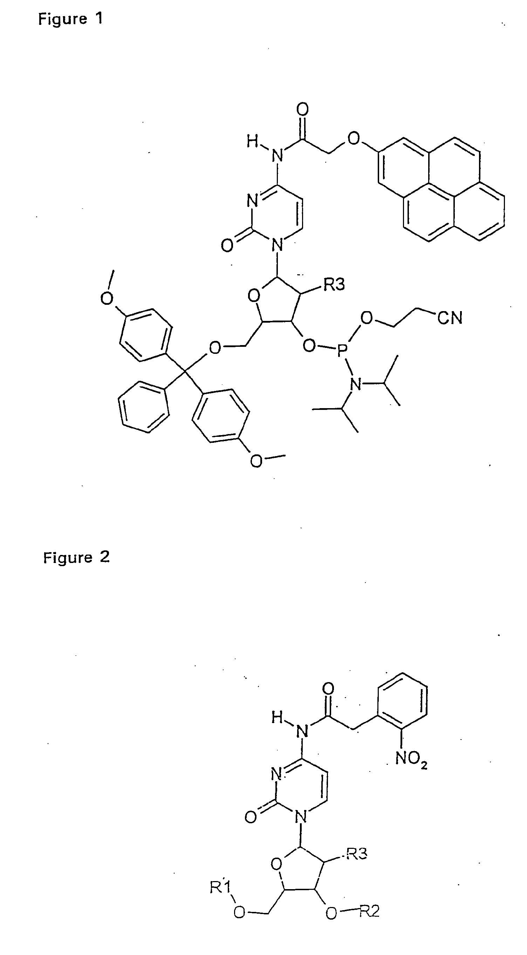

- Fig. 1.: Fluorescent, pyrene-labelled cytidine building block. The pyrene-containing nucleobase protecting group exhibits fluorescent properties and can therefore be detected by means of optical analysis procedures.
If the nucleobase aminofunctions are protected with a cleavable acyl group carrying a biotin or digoxigenin labelled system, then detection can be carried out using streptavidin or anti-digoxigenin.
- Fig. 2.: A cytidine building block with an IR detectable nitro aromatic moiety.

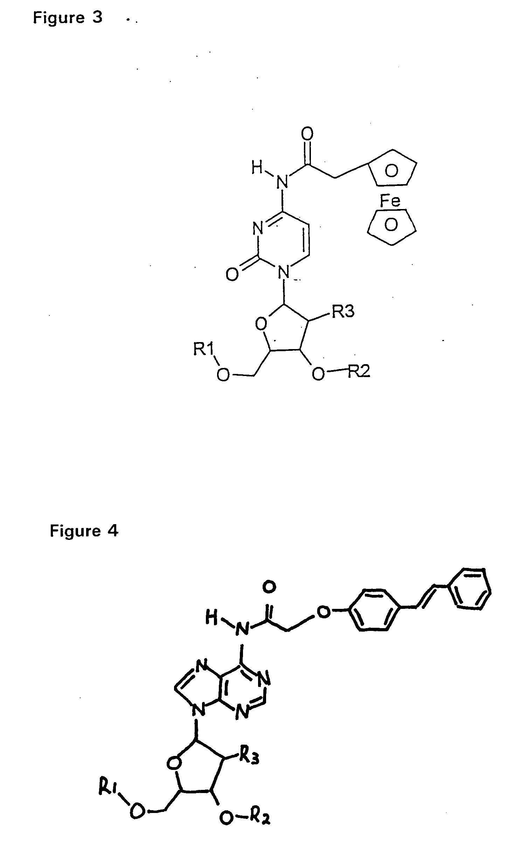

- Fig. 3.: A cytidine building block with an electrochemically detectable ferrocene moiety.
- Fig. 4.: An adenine building block with a fluorescent detectable stilbene moiety.
- Fig. 5.: Scheme of the synthesis of an adenine building block of Figure 4.

### Example 1

### Synthesis of a stilbene-labelled nucleic acid synthesis building block

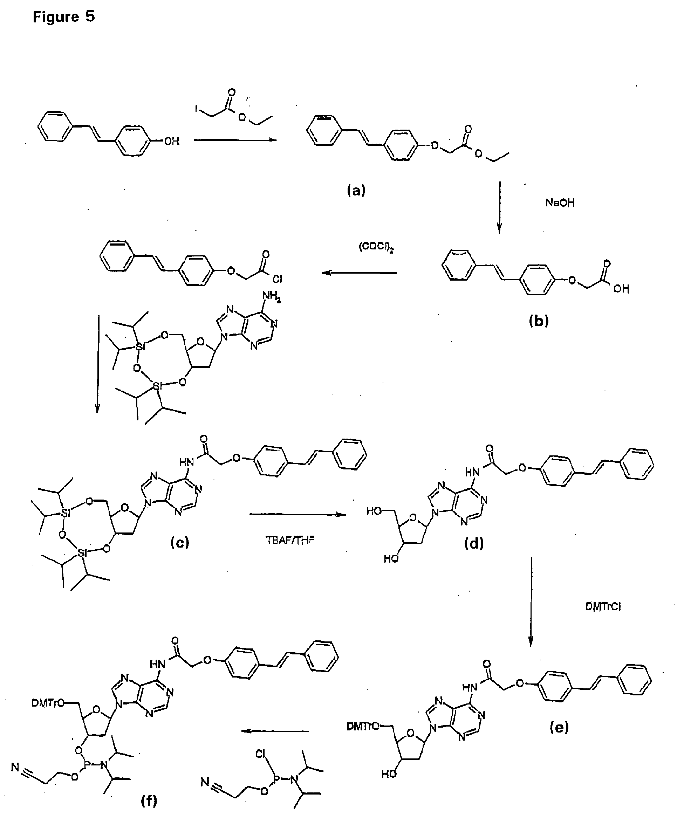

Starting from 4-hydroxystilbene the desired stilbene-labelled building block was synthesized in the following steps A) to F) according to the scheme in Figure 5:

### A) Synthesis of (4-styryl-phenoxy)-acetic acid ethyl ester (a).

550 mg sodium hydrid (60% suspension in paraffin oil) were added to a solution of 2 g trans 4 hydroxystilbene in 70 ml dry dioxane. After stirring for 1 h at room temperature 1.2 ml of iodacetic acid ethylester were added. The mixture was refluxed for 1 h and then cooled to room temperature. After filtration the solvent was removed by using a rotary evaporator and the remainder was purified by column chromatography on silica gel with hexane/acetic acid ethyl ester 2:1 as eluent.
Yield: 1,0g.

### B) Synthesis of (4-styryl-phenoxy)-acetic acid (b).

20 ml 10 Mol NaOH were added within 20 min to a solution of 1 g (4-styryl-phenoxy) acetic acid ethyl ester **(a)** in 60 ml dioxane. After stirring for 5 h the pH was adjusted to pH 2 with 10 Mol HCl. The mixture was extracted with acetic acid ethyl ester. The organic layer was separated, washed with water and dried over sodium sulfate. The solvent was evaporated by using a rotary evaporator. The remainder was dried in vacuum for 5 days with calcium chloride as dessicant.
Yield: 0.81 g.

### C) Synthesis of N6(4-styryl-phenoxy)-acetyl-4.2(-)-3',5'-O-(1,1,3,3-tetraisopropyl-1,3-disiloxanediyl) adenosine (c).

0.81 g (4-styryl-phenoxy)-acetic acid (b) was dissolved in a mixture from 15 ml dioxane and 3 ml methylene chloride. After adding 2.6 ml oxalyl chloride at 4-8°C the mixture was stirred at room temperature for 4 h. The solvents were removed by using a rotary evaporator. The remainder was dissolved in 10 ml methylene chloride. This solution was dropped at room temperature to a solution of 1.35 g 4.2 (-)-3',5'-O-(1,1,3,3-tetraisopropyl-1,3-disiloxanediyl) adenosine in 30 ml methylene chloride and 3 ml pyridine. The mixture was then stirred overnight. The solvent was removed by using a rotary evaporator and the remainder was purified by column chromatography on silica gel with toluene/acetic acid ethyl ester/methanol 4:1:1.
Yield: 1.5 g.

### D) Synthesis of N6 (4-styryl-phenoxy)-acetyl adenosine (d).

1.5 g of N6(4-styryl-phenoxy)-acetyl- 4.2 (-)-3',5'-O-(1.1.3.3-tetraisopropyl-1,3-disiloxanediyl) adenosine **(c)** were dissolved in 90 ml 1 M tetrabutylammoniumfluoride THF solution and stirred overnight. The solvent was removed by using a rotary evaporator and the remainder was purified by column chromatography on silica gel with acetic acid ethyl ester/methanol 2:1.
Yield: 0.56 g.

### E) Synthesis of 5'-(p,p'-dimethoxytrityl)- N6 (4-styryl-phenoxy)-acetyl adenosine (e).

Within 1 h a solution of 0.46 g dimethoxytritylchloride in 10 ml dry pyridine was dropped at room temperature to a solution of 0.56 g of N6 (4-styryl-phenoxy)-acetyl adenosine **(d)** in 10 ml dry pyridine. After stirring overnight the solvent was evaporated using a rotary evaporator. The residue was dissolved in 300 ml acetic acid ethyl ester and was washed with 100 ml water. The organic phase was separated and dried with sodium sulfate. After filtration the solvent was removed by using a rotary evaporator.
Purification was performed by column chromatography on silica gel. Therefore the crude product was dissolved in a methylene chloride/methanol 10:1 mixture containing 0.1% triethylamine. The solution was applied on an I = 40 cm/d = 6.9 cm column filled with silica gel 60 (0.063-0.200 mm). The product was eluated with a methylene chloride/methanol 10:1 mixture containing 0.1% triethylamine. TLC (Silicagel): methylene chloride/methanol 10:1 mixture containing 0.1% triethylamine: Rf: 0.56.
Yield: 200 mg.

### F) Synthesis of 2-Cyanoethylphosphoramidite of 5'-(p,p'-dimethoxytrityl)- N6 (4-styryl-phenoxy)-acetyl adenosine (f).

At room temperature under argon 0.56 µl of N ethyldiisopropylamine and then 73 µl chloro-2 cyanoethoxy diisopropylaminophosphane were added to a solution of 200 mg of 5'-(p,p'-dimethoxytrityl)- N6 (4-styryl-phenoxy)-acetyl adenosine **(e)** in 5 ml methylene chloride. The solvent was removed by using a rotary evaporator. The residue was dissolved in 50 ml acetic acid ethyl ester and washed two times with 10 ml 5% aqueous sodium hydrogen carbonate solution. The organic phase was separated and dried with sodium sulfate. After filtration the solvent was removed by using a rotary evaporator.

Purification was performed by column chromatography on silica gel. Therefore the crude product was dissolved in a methylene chloride/acetone/triethylamine 45:5:0.5 mixture. The solution was applied on a I = 32 cm/d = 4.5 cm column filled with silica gel 60 (0.063-0.200 mm). The product was eluated with an acetic acid ethyl ester/hexane 1:1 mixture containing 0.1% triethylamine. TLC (Silica gel, methylene chloride/acetone/triethylamine 45:5:0.5): Rf: 0.70.

NMR(^{d6}-DMSO) ¹H(300 MHz): 10.97 s[1H], 8.64 s[1H], 8.56s [1H], 6.78-7.5 m [24H], 6.50 s, broad [1H], 5.20 s[2H], 4.82 s, broad [1H], 4.16 s, broad [1H], 3.64 s [6H], 3.55 s, broad [2H], 3.64 s, broad [2H], 3.23 s, broad [2H], 3.11 m, broad [1H], 2.73 dt[2H], 2.55 m, broad [1H] 1.22 dd [12H] ³¹P(300 MHz): 148.89 d.
Yield: 120 mg.

## Claims

1. A quality control method for manufacturing biopolymer arrays comprising
(a) synthesizing a plurality of different biopolymer species on an array from monomeric or oligomeric building blocks comprising detectable protecting groups;
(b) optionally carrying out a determination of the detectable protecting groups on the array after synthesis;
(c) cleaving off the detectable protecting groups, and
(d) carrying out a determination of the detectable protecting groups on the array after cleavage; in order to determine the efficacy of deprotection.

2. The method of claim 1, wherein the detectable protecting groups are selected from fluorescent detectable protecting groups.

3. The method of claim 2, wherein the fluorescent detectable protecting groups are selected from groups comprising pyrene, dansyl, stilbene, rhodamine or coumarine moieties.

4. The method of claim 1, wherein the detectable protecting groups are selected from radioactively detectable protecting groups.

5. The method of claim 4, wherein the radioactively detectable groups are selected from groups comprising ¹⁴C, ³²P or ³H doped moieties.

6. The method of claim 1, wherein the detectable protecting groups are selected from electrochemically detectable protecting groups.

7. The method of claim 6, wherein the electrochemically detectable groups are selected from groups comprising ferrocene or phenothiazine moieties.

8. The method of claim 1, wherein the detectable protecting groups are selected from UV- or IR-detectable protecting groups.

9. The method of claim 8, wherein the UV- or IR-detectable protecting groups are selected from groups comprising aromatic nitro moieties, hydroxyl moieties, thiol, thioether or thiophenol moieties, nitrile moieties, isocyanate or halo moieties.

10. The method of claim 1, wherein the detectable protecting groups are selected from bioaffine detectable protecting groups.

11. The method of claim 10, wherein the bioaffine detectable protecting groups are selected from groups comprising biotin, digoxin or digoxigenin moieties.

12. The method of any one of claims 1-11, wherein the biopolymer species are selected from nucleic acids, nucleic acid analogues, peptides and peptide analogues.

13. The method of claim 12, wherein the biopolymer species are selected from nucleic acids and nucleic acid analogues and wherein the detectable protecting groups are coupled to nucleobases.

14. The method of claim 13 wherein the detectable protecting groups are coupled to amino groups of nucleobases.

15. The method of any one of claims 12-14, wherein the building blocks for the biopolymer synthesis are nucleotide building blocks having the general structural formulae (I) or (II): 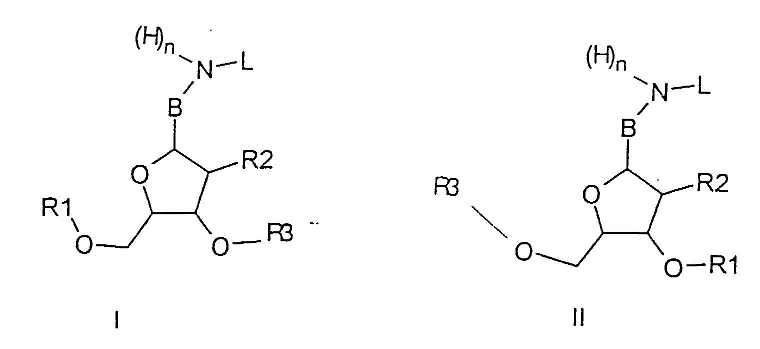 wherein R¹ is an hydroxy protecting group,
R² is -H, -(C₁-C₁₀)-alkoxy, -(C₂-C₁₀)-alkenyloxy, -(C₂-C₁₀)-alkynyloxy, -halogen, -azido, -NHR7, -SR7 or -OR7, wherein R7 is a protecting group or a reporter group,
R³ is a phosphate, an H-phosphonate or other phosphate analogue group which may contain a protecting group,
B is a nucleobase or a nucleobase analogue,
n is 0 or 1, and
L is a detectable protecting group.

16. The method of claim 15, wherein R¹ is selected from optionally substituted triphenylmethyl groups, e.g. 4,4'-dimethoxy triphenylmethyl or 4-monomethoxy triphenylmethyl, pixyl groups, photocleavable groups, e.g. p-nitrophenylpropoxy carbonyl (NPPOC) or (α-methyl)- 6-nitropiperonyloxy carbonyl (MeNPOC), and substituted silyl protecting groups, e.g. tert-butyldimethyl silyl (TBDMS) or tert-butyldiphenyl silyl (TBDPS).

17. The method of claims 15 or 16, wherein R³ is a phosphite amide group.

18. The method of claim 12 wherein R³ is the group -P(R₆)-NR₄R₅ wherein R⁴ and R⁵ are independently selected from the group consisting of -H, -(C₁-C₁₀)-alkyl, -(C₂-C₁₀)-alkenyl, -(C₆-C₂₂)-aryl, or wherein NR4R5 can form together with N a 5-6-membered heterocyclic ring, and R⁶ is selected from the group consisting of -(C₂-C₆)-alkenyloxy, -(C₂-C₆)-alkenyl, -(C₁-C₆)-alkyl, -(C₁-C₆)-alkoxy, wherein each group may contain one or several substituents selected from -halo, p-nitroaryloxy and -cyano or wherein R⁶ is -H.

19. The method of claim 18, wherein R⁶ is a 2-cyanoethyloxy group.

20. The method of any one of claims 15-19 wherein L has the structure -C(O)-R, when n = 1, or = CH-NR⁸R when n = 0, wherein R is the residue of the protecting group and R⁸ is selected from H and -(C₁-C₃)-alkyl.

21. The method of any one of claims 15-20, wherein B is selected from adenine, guanine, cytosine, aza and/or deaza analogues thereof, and analogues containing additional amino groups.

22. A nucleic acid synthesis building block having the general structural formulae (I) or (II): 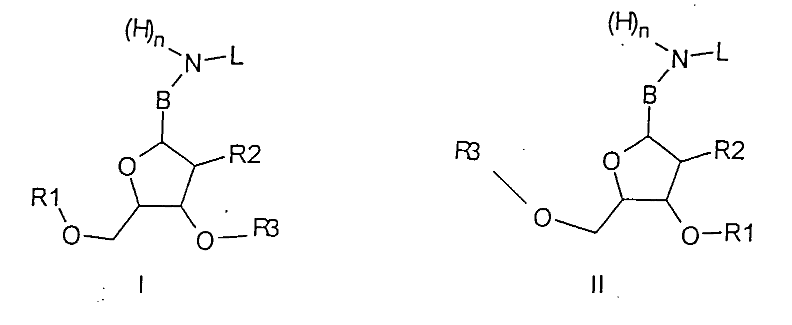 wherein R¹ is an hydroxy protecting group,
R² is -H, -(C₁-C₁₀)-alkoxy, -(C₂-C₁₀)-alkenyloxy, -(C₂-C₁₀)-alkynyloxy, -halogen, -azido, -NHR7, -SR7 or -OR7, wherein R7 is a protecting group or a reporter group,
R³ is a phosphate, an H-phosphonate or other phosphate analogue group which may contain a protecting group,
B is a nucleobase or a nucleobase analogue,
n is 0 or 1, and
L is a detectable protecting group.

23. Use of a building block of claim 22 for the production of nucleic acid arrays.

24. A reagent kit for the synthesis of nucleic acid arrays comprising at least one nucleic acid synthesis building block of claim 22.

25. The reagent kit of claim 24 comprising at least 2 nucleic acid synthesis building blocks of claim 22 each carrying different detectable protecting groups.
